# EUROPEAN PATENT SPECIFICATION

(11) **EP 1 470 205 B1**
(45) Date of publication and mention of the grant of the patent: **03.01.2007**
(21) Application number: 03742980.0
(22) Date of filing: 31.01.2003
(51) Int. Cl.: C10L 1/02, C10L 1/32

(54) **POLYAPHRON FUEL COMPOSITIONS**
POLYAPHRON-KRAFTSTOFFZUSAMMENSETZUNGEN
COMPOSITIONS DE CARBURANT A POLYAPHRON

(30) Priority: 31.01.2002 GB 0202312
(43) Date of publication of application: 27.10.2004
(73) Proprietor: Disperse Limited, Guildford, Surrey GU2 7YF (GB)
(72) Inventor: GUFFOGG, Philip, Ernest, Guildford, Surrey GU4 7NG (GB); LENON, Stephen, John, Guildford, Surrey GU4 7ES (GB); WHEELER, Derek, Alfred, Dorking, Surrey RH5 4RJ (GB)
(74) Representative: Hayes, Adrian Chetwynd
(86) International application number: PCT/GB2003/000424
(87) International publication number: WO 2003/072687

(56) References cited:
- WO-A-01/55282
- US-A- 4 486 333
- US-A- 5 646 098
- US-A1- 2001 021 494
- SEBBA F ET AL: "POSSIBLE USE OF POLYAPHRONATED HYDROCARBONS AS JET FUELS--" 193RD ACS NATL. MEET. (DENVER 4/5-10/87) ACS DIV. PET. CHEM. PREPR. V32 N.2 560-64 (APR. 1987), XP008017419 VA. TECH;WRIGHT-PATTERSON AIR FORCE BASE
- BERGERON V ET AL: "A CONTINUOUS GENERATOR FOR PRODUCING (STABLE) POLYAPHRONS" CHEM. ENG. (RUGBY) N.434 27 (MAR. 1987) ISSN: 0302-0797, XP008017408 VA. POLYTECH. INST. STATE UNIV.
- SEBBA F: "THE PREPARATION AND PROPERTIES OF POLYAPHRONS (BILIQUID FOAMS)" CHEM. IND. (LOND.) N.10 367-72 (5/21/84) ISSN: 0009-3068, XP001149037 UNIV. WITWATERSRAND

## Description

The present invention relates to polyaphron (or biliquid foam) compositions and in particular to polyaphron fuel compositions which have a high level of an organic oxygenate in the continuous phase.

Nitrogen oxides comprise a major irritant and pollutant in the atmosphere. Environmental pressures and government regulations have increased the need to reduce NOₓ emissions from internal combustion engines. One problem with using diesel-fuelled engines is that relatively high temperatures are reached during combustion, thereby increasing the tendency of nitrogen from the air, or from nitrogen compounds present in the fuel, to be oxidised to nitrogen oxides. Various methods have been preposed for reducing the production of NOₓ, including the use of catalytic convertors, the use of clean fuels, the adjustment of the engine tuning etc. These methods have not achieved a widespread use. The rate at which nitrogen oxides are produced is related to the combustion temperature and a small reduction in combustion temperature can result in a large reduction in the production of nitrogen oxides.

Emulsified fuels have been shown to provide significant advantages in relation to the control of emissions from internal combustion engines, particularly NOₓ emissions, by lowering the peak combustion temperature of the fuel.

The emulsified fuel compositions which have previously been proposed in the art are water-in-oil emulsions. For example, US Patent No. 5669938 discloses a fuel composition which consists of (i) a water-in-oil emulsion comprising a major proportion of a hydrocarbonaceous middle distillate fuel and about 1 to 40 volume percent water (ii) a co-emission and particulate reducing amount of at least one fuel-soluble organic ignition improver and other optional ingredients.

EP-B-0475620 discloses a diesel fuel composition which comprises (a) a diesel fuel, (b) 1.0 to 3.0 weight percent of water based upon the diesel fuel, (c) a cetane number improver additive in an amount of up to less than 20.0 weight percent based upon the water, and (d) 0.5 to 15 weight percent based upon the diesel fuel of a mixed surfactant system as defined.

EP-A-0561600 discloses a water-in-oil emulsion comprising a discontinuous aqueous phase containing at least one oxygen-supplying component such as ammonium nitrate, a continuous organic phase comprising at least one carbonaceous fuel, and a minor emulsifying amount of at least one emulsifier as defined.

WO-A-01/04239 discloses a process for making an aqueous hydrocarbon fuel composition comprising: (A) mixing a normally liquid hydrocarbon fuel and at least one chemical additive to form a hydrocarbon fuel-additive mixture; and (B) mixing the hydrocarbon fuel-additive mixture with water under high shear mixing conditions in a high shear mixer to form said aqueous hydrocarbon fuel composition, said aqueous hydrocarbon fuel composition including a discontinuous aqueous phase, said discontinuous aqueous phase being comprised of aqueous droplets having a mean diameter of 1.0 micrometres or less.

WO-A-00/15740 discloses an emulsified water-blended fuel composition comprising (A) a hydrocarbon boiling in the gasoline or diesel range, (B) water, (C) a minor emulsifying amount of at least one fuel soluble salt made by reacting an acylating agent having about 16 to 500 carbon atoms with ammonia or an amine, and (D) about 0.001 to 15% by weight of the water-blended fuel composition of a water-insoluble amine salt distinct from component (C).

The water-in-oil emulsified fuels of the prior art suffer from some significant disadvantages.

First, they need to contain high levels of emulsifiers to generate stable systems. Investigations have shown that the effectiveness of the reduction in NOₓ-emissions depends upon the stability of the emulsion. For a reliable operation it is necessary to create an emulsion with a minimum stability of more than 13 minutes. Secondly, because the continuous phase is oil and not water they do not present the ignition source with the water first and therefore do not cool the charge immediately, resulting in the less efficient use of the water contained in the emulsion to give a cleaner burn. Thirdly, there is a limit to the level of organic oxygenate compounds that can be included in these systems because of stability issues. These fuels suffer from a sluggish drive compared to a standard fuel which is a particular disadvantage in situations where power-loss can be unacceptable, such as non-static engines.

Biliquid foams are known in the art in which small droplets of a non-polar liquid such as an oil are encapsulated in a surfactant-stabilized film of a hydrogen bonded liquid, such as water, and separated from one another by a thin film of the hydrogen bonded liquid. The water or other hydrogen bonded liquid thus forms the continuous phase in biliquid foam compositions.

US-A-4486333 discloses a method for the preparation of biliquid foam compositions which may comprise the non-polar liquid in a total amount of about 60% to about 98% by volume, the hydrogen bonded liquid constituting the balance. The non-polar liquid may comprise a petroleum derivative, paraffin or a liquid halogenated hydrocarbon. The biliquid fuel composition prepared comprising 96% by volume methanol and 4% by volume water had a limited stability of only several days.

Accordingly, there is a need to generate cleaner fuels with lower emissions, especially in the diesel fuel sector. There is also a requirement from the consumer not to compromise on the power output of the fuel. There is also a desire for safer fuels and, in particular, fuels with a reduced or more acceptable odour.

We have now developed a biliquid fuel composition which meets these requirements and which comprises an aqueous continuous phase with a high alcohol level. This results in a more acceptable odour, lower emissions and a safer fuel with no loss of power. The bifluid fuel composition is safer to the environment as it has a polar continuous phase, and therefore in the case of spillage it can be washed away as it is water disperisble. The presence of organic oxygenates will also lower the freezing point compared to a fuel in water only composition. Furthermore, the bifluid fuel composition which we have developed has improved storage stability as compared with the prior art compositions disclosed in US-A-4486333.

Accordingly, the present invention provides a fuel composition which comprises a biliquid foam consisting of from 10% to 97.5% by weight of non-coalescing droplets of a non-polar liquid comprising a petroleum derivative, paraffin or a liquid halogenated hydrocarbon and from 2 to 87% by weight of a continuous phase polar liquid comprising a C₁-C₃ alcohol, a C₄ alcohol containing at least two hydroxy groups, or ethylene glycol, or mixtures thereof, in an amount of from 60% to 100% by weight thereof, wherein the biliquid foam is stabilized with an amount of from 0.5 to 3.0% by weight based on the total formulation of a surfactant which is selected from castor oil/poly(alkylene glycol) adducts containing from 20 to 50 alkoxy groups, or hydrogenated castor oil/poly(alkylene glycol) adducts containing from 20 to 60 alkoxy groups, or mixtures thereof.

The biliquid fuel of the present invention preferably comprises from 60% to 90% by weight of the fuel, more preferably from 75% to 85% by weight of the fuel. The preferred fuel for use in the present invention is diesel, gasoline or kerosene.

The polar liquid preferably comprises from 50% to 99% by weight of the C₁-C₄ alcohol or ethylene glycol, or mixtures thereof. The balance of the polar liquid is preferably water, in particular deionized water. The preferred polar liquids for use in the invention are methanol or ethanol, or mixtures thereof.

The particular classes of surfactant used in the present invention have been selected for use because of their ability to assist in the preparation of the biliquid fuel compositions and because they impart long term stability (e.g. 30 to 90 days) upon the majority of the biliquid fuel compositions of the present invention prepared using them.

The preferred classes of surfactants for use in the present invention are hydrogenated castor oil/polyethylene glycol adducts containing from 25 to 50 ethoxy groups, more preferably 40 to 45 ethoxy groups or castor oil/polyethylene glycol adducts containing from 25 to 45 ethoxy groups.

It will be understood by those skilled in the art that the choice of surfactant will also depend upon the particular fuel and the particular polar liquid and the amount thereof which are used in the preparation of the bifluid fuels.

Polyisobutylene succinimide esters or polyisobutylene succinimide amines or Mannich bases, known to those skilled in the art as ashless dispersants, may be used as co-surfactants in the present invention.

The preferred amount of surfactant for use in the present invention is about 1% by weight based on the total formulation.

The biliquid fuel compositions of the present invention may also contain other additives such as preservatives (for instance to prevent microbiological spoilage), corrosion inhibitors, lubricity improves cleaning/detergent additives, urea and other additives These additives may be included in the non-polar liquid or the continuous phase to enhance the performance and address legislative requirements.

It will be understood that the inclusion of these additives will be at the levels and with the type of materials which are found to be effective and useful. Care needs to be taken in the choice and amount of these additives to prevent compromise to the other performance advantages of the present invention.

Methods of producing biliquid foams are described in US-A-4486333 involving the preliminary formation of a gas foam in order to provide a sufficiently large surface area on which the biliquid foam can subsequently be formed. It has been found that the prior formation of a gas foam is not required to manufacture a stable biliquid foam, provided that a suitable stirring mechanism is provided in the manufacturing vessel. An important aspect of the present invention is the ability to manufacture biliquid foams suitable for use as a fuel composition without the preliminary formation of gas foam, by the use of a tank incorporating a suitable stirring mechanism.

Such an apparatus comprises a tank provided with a stirrer in which the stirrer blade breaks the interface between the liquid and air. A delivery device is provided through which the oil phase (non-polar liquid), which will comprise the internal phase of the dispersion is delivered to the tank. The design of the delivery device is such that the rate of addition of the internal phase fluid can be controlled and varied during the production process. A feature of the production process is that the internal (oil) phase is added to the stirred aqueous phase slowly at first until sufficient droplets have been formed to constitute a large, additional surface area for the more rapid formation of new droplets. At this point, the rate of addition of the oil phase may be increased.

The present invention will be further described with reference to the following Examples.

### EXAMPLES 1 to 24 and

### Comparative EXAMPLES A to C

Biliquid fuel composition were prepared in 50g batches by adding to a 250ml squat beaker of 67ml internal diameter a premixed and homogeneous methanol, ethanol and/or ethylene glycol-water mixture and surfactant. A 4 blade axial flow impeller of diameter 55mm was lowered into the beaker to agitate the aqueous phase ensuring that the impeller cut the air/liquid interface and was rotated at 200rpm. The fuel was slowly added dropwise to the aqueous phase over a ten to fifteen minute period of time. After all of the fuel had been added, the mixture was stirred for a further fifteen minutes to form the desired biliquid fuel compositions.

The results obtained for compositions comprising 80% by weight diesel, 1% by weight surfactant and 19% by weight of alcohol-water mixture are given in Table 1 below. The percentage column in Table 1 for the alcohol refers to the percentage in the alcohol-water mixture.

In Comparative Examples A, B and C, the compositions comprise the surfactants Protachem CAH-16, Etocas 15 and Protachem CA-200, which do not fall within the scope of the present invention.

Where stability is given in days this is the point at which instability occurred. Where it is stated with '+' after the number, this means that the sample has not been observed to be unstable in that time and storage is ongoing. All samples were monitored for storage stability at ambient temperature. (ca.20-25°C).

**TABLE 1**

| **Ex. No.** | **Alcohol** | **(%)** | **Surfactant** | **Stability in days** | **Comments on stability.** |
|---|---|---|---|---|---|
| 1 | Ethanol | 70 | Croduret 50 | 91+ | Only slight oil on surface. |
| 2 | Ethanol | 80 | Croduret 50 | 91+ | Some foaming and slight oil on surface. |
| 3 | Ethanol | 85 | Croduret 50 | 85+ | Some oil on surface. After 81 days some decomposition on the upper layer is noticed. |
| 4 | Ethanol | 90 | Croduret 50 | 8 | After 8 days sample begins to show some signs of decomposition. |
| 5 | Methanol | 70 | Croduret 50 | 90+ | Only slight oil on surface. |
| 6 | Methanol | 80 | Croduret 50 | 90+ | Some oil on surface. |
| 7 | Methanol | 90 | Croduret 50 | 90+ | Some oil on surface. |
| 8 | Methanol | 100 | Croduret 50 | 44 | After 44 days the amount of oil begins to increase on surface. After that sample begins slowly to decompose. |
| 9 | Ethylene- glycol | 70 | Croduret 50 | 82+ | No changes. |
| 10 | Ethylene- glycol | 80 | Croduret 50 | 82+ | Some foaming, no other changes. |
| 11 | Ethylene- glycol | 90 | Croduret 50 | 82+ | No changes. |
| 12 | Ethylene- glycol | 95 | Croduret 50 | 82+ | Some foaming, slight oil on surface. |
| 13 | Ethanol | 35 | Croduret 50 | 69+ | No changes. |
| | Methanol | 35 | | | |
| 14 | Ethanol | 35 | Croduret 50 | 69+ | No changes. |
| | Ethylene glycol | 35 | | | |
| 15 | Ethanol | 70 | Croduret 25 | 70+ | Some oil on surface. |
| 16 | Ethanol | 70 | Croduret 25* | 70+ | Slight oil on surface. |
| 17 | Ethanol | 70 | Croduret 25** | 70+ | Some oil on surface. |
| 18 | Ethanol | 70 | Croduret 40LD | 15+ | No changes. |
| 19 | Ethanol | 70 | Protachem CAH-60 | 15+ | No changes. |
| 20 | Ethanol | 70 | Etocas 25 | 3 | After three days, the amount of free oil begins to increase. |
| 21 | Ethanol | 70 | Protachem CA-30 | 3 | After 6 days a lot of free oil on surface, and some decomposition. |
| 22 | Ethanol | 70 | Etocas 35 | 48 | After 48 days decomposition of sample leading to free oil and instability. |
| 23 | Ethanol | 70 | Etocas 40 | 18 | After 18 days gradual decomposition begins. |
| 24 | Ethanol | 70 | Protachem CA-40 | 60+ | Beginning to decompose gradually, but still stable overall. |
| Comp A | Ethanol | 70 | Protachem CAH-16 | < 1 | Begins to decompose gradually. |
| Comp B | Ethanol | 70 | Etocas 15 | few min | Product is stable for 5-10 minutes. |
| Comp C | Ethanol | 70 | Protachem CA-200 | few min | Stable only for a few minutes. |

### EXAMPLE 25

The procedure of Example 1 was repeated substituting unleaded petrol for the diesel fuel. The resulting biliquid fuel was stable for 64 days after which some instability was seen.

### EXAMPLE 26

The procedure of Example 1 was repeated substituting kerosene for the diesel fuel. The resulting biliquid fuel was stable for 43 days after which the sample began to show some signs of instability.

### EXAMPLE 27

The procedure of Example 1 was repeated for a composition comprising 75% by weight of diesel fuel, 1% by weight of Croduret 50 and 24% by weight of an ethanol-water mixture comprising 70% by weight of ethanol. The resulting bifluid fuel was stable for over 123 days.

### EXAMPLES 28 to 32

The procedure of Example 1 was repeated for compositions comprising 85% by weight of diesel fuel, 1% of a surfactant and 14% by weight of an alcohol-water mixture. The results are given in Table 2 below. The percentage column in Table 1 for the alcohol refers to the percentage in the alcohol-water mixture.

**TABLE 2**

| **Ex. No.** | **Alcohol** | **(%)** | **Surfactant** | **Stability in days** | **Comments on stability.** |
|---|---|---|---|---|---|
| 28 | Ethanol | 70 | Croduret | 126+ | Stable. |
| | | | 50 | | |
| 29 | Isopropyl- | 70 | Croduret | 125+ | Stable, with slight oil on surface |
| | alcohol | | 50 | | |
| 30 | Ethanol | 70 | Croduret | 169+ | Stable, with slight oil on surface |
| | | | 25 | | |
| 31 | Ethanol | 70 | Protachem | 126+ | Stable |
| | | | CAH-60 | | |
| 32 | Ethanol | 70 | Croduret | 126+ | Stable |
| | | | 40 LD | | |

| | | | | | |
|---|---|---|---|---|---|
| Footnotes to Tables 1 and 2 * The surfactant was added to the oil phase ** The surfactant was included in both phases (50:50) | | | | | |

Croduret 25, Croduret 40LD and Croduret 50 are Trade Names for hydrogenated castor oil/polyethylene glycol adducts available from Croda Chemicals Limited where the suffixes 25, 40LD and 50 refer to increasing numbers of ethylene oxide groups in the polyethylene oxide chain.

Etocas 15, Etocas 25, Etocas 35 and Etocas 40 are Trade Names for castor oil/polyethylene glycol adducts available from Croda Limited where the suffixes 15, 25, 35 and 40 refer to the number of ethylene oxide groups in the polyethylene oxide chain.

Protachem CA-30, Protachem CA-40 and Protachem CA-200 are Trade Names for castor oil/polyethylene glycol adducts available from Protameen Chemicals Inc. where the suffixes CA-30, CA-40 and CA-200 refer to the number of ethylene oxide groups in the polyethylene oxide chain.

Protachem CAH-16 and CAH-60 are a Trade Names for a hydrogenated castor oil/polyethylene glycol adducts available from Protameen Chemicals Inc. where the suffix CAH-16 and CAH-60 refers to the number of ethylene oxide groups in the polyethylene oxide chain.

### Comparative EXAMPLE D

The teaching of Example 7 of US Patent No. 4486333 was repeated. A biliquid fuel composition was prepared by making a 0.5% solution of a silicone block copolymer L5614 in 96% methanol, foaming 5 ml of this solution and gradually adding thereto 45ml of kerosene containing 0.1% Tergitol 15-S-3 with vigorous shaking after each addition of the oil phase.

The sample inverted after the addition of 75% (about 34 ml) of the oil phase.

### EXAMPLE 33

The teaching of Example 7 of US Patent No. 4486333 was modified using as the sole surfactant 1% Croduret 50 in 96% methanol.

A bifluid fuel composition was prepared by stirring 5ml of the methanol solution and gradually adding thereto 45ml of kerosene using the stirring method as described in Example 1. After all of the fuel had been added, the mixture was stirred at 300 rpm for a further 15 minutes to form the bifluid fuel composition.

The formulation remained stable for three weeks.

### EXAMPLES 34 TO 37

Examples 35 to 38 illustrate the preparation of bifluid fuel compositions using isopropyl alcohol/water and isopropylalcohol/ethanol/water mixtures as the polar liquid.

The bifluid fuels were prepared according to the teaching of Example 1 but with an addition time for the oil phase of 20 minutes and an additional stirring time of 20 minutes after completion of the oil addition. Examples 36 and 37 were prepared as 25g batches. The results obtained for compositions comprising 80% by weight of diesel, 1% of surfactant and 19% of an alcohol/water mixture are given in Table 3 below. The percentage column in Table 3 for the alcohol refers to the percentage in the alcohol-water mixture.

**TABLE 3**

| **Ex. No.** | **Alcohol** | **(%)** | **Surfactant** | **Stability in days** | **Comments on stability.** |
|---|---|---|---|---|---|
| 34 | Isopropyl alcohol | 70 | Croduret 50 | 16 | Slight decomposition in upper layer after 5 days leading to progressive deterioration. |
| 35 | Isopropyl alcohol | 65 | Croduret 50 | 59 | Slight decomposition in upper layer at first after 28 days leading to progressive decomposition. |
| 36 | Isopropyl alcohol | 35 | Croduret 50 | 11 | Free oil on the surface of sample at first followed by decomposition. |
| | Ethanol | 35 | | | |
| 37 | Isopropyl alcohol | 42 | Croduret 50 | 11 | Separation into two layers giving rapid decomposition. |
| | Ethanol | 28 | | | |

### EXAMPLE 38 TO 42

Examples 38 to 42 illustrate the preparation of bifluid fuel compositions using butylene glycol/water, butylene glycol/ethylene glycol/water or propylene glycol/water mixtures as the polar liquid.

The bifluid fuels were prepared according to the teaching of Example 1 but with an addition time for the oil phase of 20 minutes and an additional stirring time of 20 minutes after completion of the oil addition. The results obtained for compositions comprising 80% by weight of diesel, 1% of surfactant and 19% of the glycol/water mixture are given in Table 4 below. The percentage column in Table 4 for the glycol refers to the percentage in the glycol-water mixture.

**TABLE 4**

| **Ex. No.** | **Alcohol** | **(%)** | **Surfactant** | **Stability in days** | **Comments on stability.** |
|---|---|---|---|---|---|
| 38 | Butylene glycol | 35 | Croduret 50 | 40+ | Stable. |
| | Ethylene glycol | 35 | | | |
| 39 | Butylene glycol | 50 | Croduret 50 | 40+ | Stable. |
| | Ethylene glycol | 20 | | | |
| 40 | Butylene glycol | 60 | Croduret 50 | 40+ | Stable. |
| 41 | Butylene glycol | 70 | Croduret 50 | 39+ | Stable. |
| 42 | Propylene glycol | 70 | Croduret 50 | 39+ | Stable. |

### EXAMPLE 43

A bifluid fuel composition was prepared using the stirring method as described in Example 1 as a 25g sample using 80% by weight of kerosene Jet A1 fuel, 1% by weight Croduret 50 and 19% by weight of a 96% methanol - 4% water (by weight) mixture. The addition of the oil phase was carried out over a 20 minute period and the composition was stirred for an additional 20 minutes after completion of the oil addition.

The resulting composition was stable for 32 days after which time there was decomposition leading to progressive deterioration.

## Claims

1. A fuel composition which comprises a biliquid foam consisting of from 10% to 97.5% by weight of non-coalescing droplets of a non-polar liquid comprising a petroleum derivative, paraffin or a liquid halogenated hydrocarbon and from 2 to 87% by weight of a continuous phase polar liquid comprising a C₁-C₃ alcohol, a C₄ alcohol containing at least two hydroxy groups, or ethylene glycol, or mixtures thereof, in an amount of from 60% to 100% by weight thereof, wherein the biliquid foam is stabilized with an amount of from 0.5 to 3.0% by weight based on the total formulation of a surfactant which is selected from castor oil/poly(alkylene glycol) adducts containing from 20 to 50 alkoxy groups, or hydrogenated castor oil/poly(alkylene glycol) adducts containing from 20 to 60. alkoxy groups, or mixtures thereof.

2. A fuel composition as claimed in claim 1 wherein the amount of surfactant is about 1% by weight based on the total formulation.

3. A fuel composition as claimed in claim 1 or claim 2 wherein the surfactant comprises a hydrogenated castor oil/polyethylene glycol adduct containing from 25 to 50 ethoxy groups.

4. A fuel composition as claimed in claim 3 wherein the hydrogenated castor oil/polyethylene glycol adduct contains from 40 to 45 ethoxy groups.

5. A fuel composition as claimed in claim 1 or claim 2 wherein the surfactant comprises a castor oil/poly(alkylene glycol) adduct containing 25 to 45 ethoxy groups.

6. A fuel composition as claimed in any one of the preceding claims wherein the polar liquid comprises from 70% to 99% by weight of the C₁-C₄ alcohol or ethylene glycol.

7. A fuel composition as.claimed in claim 6 wherein the balance of the polar liquid comprises water.

8. A fuel composition as claimed in any one of the preceding claims wherein non-polar liquid comprises from 60% to 90% by weight of the biliquid foam.

9. A fuel composition as claimed in claim 8 wherein the non-polar liquid comprises from 75% to 85% by weight of the bilquid foam.

10. A fuel composition as claimed in any one of the preceding claims wherein the non-polar liquid comprises diesel, gasoline or kerosene fuel.

11. A fuel composition as claimed in any one of the preceding claims wherein the C₁-C₄ alcohol is methanol or ethanol or a mixture thereof.

## Patentansprüche

1. Brennstoffzusammensetzung, welche umfasst einen biliquiden Schaum bestehend aus von 10 Gew.-% bis 97,5 Gew.-% nichtkoaleszierender Tropfen einer unpolaren Flüssigkeit umfassend ein Petroleumderivat, Paraffin oder einen flüssigen halogenierten Kohlenwasserstoff, und von 2 bis 87 Gew.-% einer polaren Flüssigkeit mit kontinuierlicher Phase umfassend einen C₁-C₃-Alkohol, einen C₄-Alkohol enthaltend wenigstens zwei Hydroxygruppen oder Ethylenglykol oder Mischungen davon in einer Menge von 60 Gew.-% bis 100 Gew.-% davon, wobei der biliquide Schaum stabilisiert ist mit einer Menge einer oberflächenaktiven Substanz von 0,5 bis 3,0 Gew.-% basierend auf der gesamten Formulierung, wobei die oberflächenaktive Substanz ausgewählt ist aus Rizinusöl/Poly(alkylenglykol)-Addukten enthaltend von 20 bis 50 Alkoxygruppen oder hydrierten Rizinusöl/Poly(alkylenglykol)-Addukten enthaltend von 20 bis 60 Alkoxygruppen oder Mischungen davon.

2. Brennstoffzusammensetzung wie in Anspruch 1 beansprucht, wobei die Menge der oberflächenaktiven Substanz etwa 1 Gew.-% basierend auf der gesamten Formulierung beträgt.

3. Brennstoffzusammensetzung wie in Anspruch 1 oder Anspruch 2 beansprucht,
wobei die oberflächenaktive Substanz ein hydriertes Rizinusöl/Polyethylenglykoladdukt enthaltend von 25 bis 50 Ethoxygruppen umfasst.

4. Brennstoffzusammensetzung wie in Anspruch 3 beansprucht, wobei das hydrierte Rizinusöl/Polyethylenglykoladdukt von 40 bis 45 Ethoxygruppen enthält.

5. Brennstoffzusammensetzung wie in Anspruch 1 oder Anspruch 2 beansprucht,
wobei die oberflächenaktive Substanz ein Rizinusöl/Poly(alkylenglykol)-Addukt enthaltend 25 bis 45 Ethoxygruppen umfasst.

6. Brennstoffzusammensetzung wie in einem der vorhergehenden Ansprüche beansprucht, wobei die polare Flüssigkeit von 70 Gew.-% bis 99 Gew.-% des C₁-C₄-Alkohols oder des Ethylenglykols umfasst.

7. Brennstoffzusammensetzung wie in Anspruch 6 beansprucht, wobei der Rest der polaren Flüssigkeit Wasser umfasst.

8. Brennstoffzusammensetzung wie in einem der vorhergehenden Ansprüche beansprucht, wobei die unpolare Flüssigkeit von 60 Gew.-% bis 90 Gew.-% des biliquiden Schaums umfasst.

9. Brennstoffzusammensetzung wie in Anspruch 8 beansprucht, wobei die unpolare Flüssigkeit von 75 Gew.-% bis 85 Gew.-% des biliquiden Schaums umfasst.

10. Brennstoffzusammensetzung wie in einem der vorhergehenden Ansprüche beansprucht, wobei die unpolare Flüssigkeit Diesel-, Benzin- oder Kerosinbrennstoff umfasst.

11. Brennstoffzusammensetzung wie in einem der vorhergehenden Ansprüche beansprucht, wobei der C₁-C₄-Alkohol Methanol oder Ethanol oder eine Mischung davon ist.

## Revendications

1. Composition de carburant qui comprend une mousse biliquide constituée de 10 % à 97,5 % en poids de gouttelettes non coalescentes d'un liquide non polaire comprenant un dérivé de pétrole, une paraffine ou un hydrocarbure halogéné liquide, et 2 à 87 % en poids d'un liquide polaire de phase continue comprenant un alcool en C₁ à C₃, un alcool en C₄ contenant au moins deux groupes hydroxy ou de l'éthylèneglycol, ou des mélanges d'entre eux, en une quantité de 60 % à 100 % en poids, dans laquelle la mousse biliquide est stabilisée avec une quantité de 0,5 à 3,0 % en poids, par rapport à la formulation totale, d'un agent tensio-actif qui est choisi parmi les produits d'addition d'huile de ricin/poly(alkylèneglycol) contenant 20 à 50 groupes alkoxy, ou les produits d'addition d'huile de ricin hydrogénée/poly(alkylèneglycol) contenant 20 à 60 groupes alkoxy, ou leurs mélanges.

2. Composition de carburant selon la revendication 1, dans laquelle la quantité d'agent tensio-actif est d'environ 1 % en poids par rapport à la formulation totale.

3. Composition de carburant selon la revendication 1 ou la revendication 2, dans laquelle l'agent tensio-actif comprend un produit d'addition d'huile de ricin hydrogénée/polyéthylèneglycol contenant 25 à 50 groupes éthoxy.

4. Composition de carburant selon la revendication 3, dans laquelle le produit d'addition d'huile de ricin hydrogénée/polyéthylèneglycol contient 40 à 45 groupes éthoxy.

5. Composition de carburant selon la revendication 1 ou la revendication 2, dans laquelle l'agent tensio-actif comprend un produit d'addition d'huile de ricin/poly-(alkylèneglycol) contenant 25 à 45 groupes éthoxy.

6. Composition de carburant selon l'une quelconque des revendications précédentes, dans laquelle le liquide polaire comprend 70 % à 99 % en poids de l'alcool en C₁ à C₄ ou d'éthylèneglycol.

7. Composition de carburant selon la revendication 6, dans laquelle le reste du liquide polaire consiste en eau.

8. Composition de carburant selon l'une quelconque des revendications précédentes, dans laquelle le liquide non polaire constitue 60 % à 90 % en poids de la mousse biliquide.

9. Composition de carburant selon la revendication 8, dans laquelle le liquide non polaire constitue 75 % à 85 % en poids de la mousse biliquide.

10. Composition de carburant selon l'une quelconque des revendications précédentes, dans laquelle le liquide non polaire comprend du carburant diesel, de l'essence ou du kérosène.

11. Composition de carburant selon l'une quelconque des revendications précédentes, dans laquelle l'alcool en C₁ à C₄ est le méthanol ou l'éthanol ou un mélange d'entre eux.
